Europäisches Patentamt

European Patent Office

Office européen des brevets

(19)

(11) Veröffentlichungsnummer: **0 226 568**
**A1**

## (12) EUROPÄISCHE PATENTANMELDUNG

(21) Anmeldenummer: 86890281.8

(22) Anmeldetag: 15.10.86

(51) Int. Cl.⁴: **A 61 N 1/04**

(30) Priorität: 08.11.85 AT 3257/85

(43) Veröffentlichungstag der Anmeldung:
24.06.87 Patentblatt 87/26

(84) Benannte Vertragsstaaten:
AT BE CH DE ES FR GB GR IT LI LU NL SE

(71) Anmelder: **HEPAX Limited**
**Meierhofstrasse 325**
**FL-9495 Triesen (LI)**

(72) Erfinder: **Povse, Walter**
**Kapellengasse 7/1/18**
**A-2514 Traiskirchen (AT)**

(74) Vertreter: **Krause, Walter, Dr. Dipl.-Ing.**
**Postfach 200 Singerstrasse 8**
**A-1014 Wien (AT)**

(54) Elektrode für die Elektrotherapie.

(57) Die Erfindung betrifft eine Elektroden für die Elektrotherapie, mit einer leitenden, hautfreundlichen Grundschicht (1) und einem an der der Hautoberfläche abgewandten Seite der Grundschicht angeordneten stromverteilendes Element, welches mit einem Kabel leitend verbindbar ist. Zur besseren homogenen Stromverteilung und zur Gewährleistung, daß sich die Elektrode möglichst ideal an den Körper anschmiegt ist vorgesehen, daß das elektrisch leitende Element aus einer dünnen Metallfolie besteht, die zusammen mit dem abisolierten Ende in der Grundschicht eingebettet ist, sowie daß Bereiche vorhanden sind, in welchen die Klebeschicht der Deckschicht zur Grundschicht durchdringt.

*Fig. 1*

EP 0 226 568 A1

Bundesdruckerei Berlin

**Beschreibung**

Elektrode für die Elektrotherapie

Die Erfindung bezieht sich auf eine Elektrode für Elektrotherapie, mit einer leitenden, selbsthaftend an der Hautoberfläche eines Patienten anbringbaren Grundschicht, mit einem Kabel, dessen abisoliertes Ende mit einem auf der Grundschicht aufliegenden, elektrisch leitenden Element verbunden ist, sowie mit einer Deckschicht.

Eine Elektrode der eingangs genannten Art ist aus der US-PS 4 248 247 bekannt. Diese weist eine rechteckige Isolierschicht auf, welche durch eine Mittellinie in zwei Hälften unterteilt ist. Auf einer Hälfte wird auf das abisolierte Ende eines Kabels ein elektrisch leitendes Element aufgeklebt. Die andere Hälfte weist eine rechteckige Aussparung auf, die in ihrer Größe dem leitenden Element entspricht, welches symmetrisch zur Aussparung angeordnet ist, sodaß beim Zusammenfalten der Isolierschicht entlang der Mittellinie das leitende Element genau in die Aussparung paßt. Schließlich wird als Grundschicht, welche mit der Hautoberfläche in Kontakt tritt, eine leitende selbsthaftende Schicht angebracht, die mit dem leitenden Element in großflächigem Kontakt steht.

Die Isolierschicht besteht aus geschäumtem Polyäthylen und weist eine Dicke von ca. 1,5 mm auf. Das elektrisch leitende Element ist aus Gummi mit eingelagerten leitenden Partikeln aufgebaut und weist eine ähnliche Dicke auf. Durch das Zusammenfalten der Isolierschicht entsteht somit eine mehrschichtige relativ dicke Elektrode, die sich nicht in idealer Weise an die Körperoberfläche anschmiegt. Bedingt durch Alterung vergrößert sich der elektrische Widerstand der leitenden Gummischicht, sodaß dann nicht mehr von einer homogenen Stromverteilung über die gesamte Elektrodenoberfläche gesprochen werden kann.

Aus der EP-Al 2 059 ist eine Elektrode bekannt, welche eine leitende Schicht mit Silberpartikel aufweist, wobei das abisolierte Ende der elektrischen Leitung nur in einem kleinen Bereich auf der leitenden Schicht, welche auch mit der Hautoberfläche in Kontakt gebracht wird, aufliegt. Das aufgefächerte abisolierte Ende der Leitung wird durch eine Abdeckung festgehalten und isoliert. Nachteilig dabei ist die inhomogene Stromverteilung durch die einseitige Kontaktierung der leitenden Schicht, wodurch es zu lokalen Flußkonzentrationen - sogenannten "hot spots" - kommen kann.

Eine Stimulationselektrode, welche eine relativ gleichmässige Stromverteilung innerhalb der Elektrode aufweist, wird in der US-PS 4 125 110 beschrieben, wobei für guten Elektrodenkontakt mit der Hautoberfläche des Patienten gesorgt ist. Die Grundschicht dieser Elektrode weist hauptsächlich folgende Inhaltsstoffe auf.

Ein hydrophiles Polysaccharid, beispielsweise das Naturprodukt Karaya, elektrolytische Salze, Wasser und einen höherwertigen Alkohol. Die genannte Grundschicht ist einerseits biegsam und gleicht sich den Unebenheiten der Hautoberfläche gut an, sie weist jedoch andererseits genügend Festigkeit auf, sodaß eine gewisse Formbeständigkeit gewährleistet ist. Auf der von der Hautoberfläche abgewandten Seite der Grundschicht befindet sich ein leitendes stromverteilendes Element, durch welche eine homogene Stromverteilung über die gesamte Elektrode erreicht wird. Dadurch werden Inhomogenitäten im Stromfluß, die zu lokalen Flußkonzentrationen führen und in diesen -"hot spots" genannten Bereichen zu unangenehmen Sensationen bzw. Verbrennungen führen können, vermieden. Im leitenden Element ist ein elektrischer Kontaktknopf eingenietet und so mechanisch und elektrisch leitend mit diesem verbunden. Dieser Kontaktknopf ist über das an einem Kabel befestigte Gegenstück mit dem Therapiegerät verbindbar.

Nachteilig ist bei dieser Ausführung, daß die bei der Herstellung des elektrischen Kontaktes, d.h. beim Zusammenfügen von Kontaktknopf und seinem Gegenstück notwendigen Kräfte, zu einem allmählichen Durchdrücken der elastischen Grundschicht führen, wobei zusätzlich die Verbindung des Kontaktknopfes zum leitenden Element beschädigt wird. Störend wirkt der elektrische Kontaktknopf, auch bei am Patienten angebrachten Elektroden, vor allem bei Elektroden mit kleinem Durchmesser, da über den Kontaktknopf auf die Elektrode schon bei geringer Zugbelastung ein Kippmoment erreicht wird, durch welches die Elektrode verrutscht oder überhaupt der Hautkontakt verloren geht. Außerdem kann ein Durchdrücken des Kontaktknopfes durch die Grundschicht der Elektrode auch durch den Patienten selbst erfolgen, wenn er mit Elektroden versehene Körperstellen zu sehr belastet, beispielsweise durch Darauflegen, wodurch trotz stromverteilendem Element "hot spots" auftreten können.

Ein weiterer Nachteil ist die ungenügende Fixierung des stromverteilenden Elementes auf der Grundschicht sowie dessen unzureichende Isolierung auf der der Hautoberfläche abgewandten Seite.

Aufgabe der vorliegenden Erfindung ist es, eine Elektrode der eingangs genannten Art so auszubilden, daß die genannten Nachteile vermieden werden und daß insbesondere eine Elektrode einfacher Bauweise geschaffen wird, welche dünn und damit anschmiegsam ist, wobei das elektrisch lei tende Element für eine homogene Stromverteilung sorgt und ausreichend fixiert ist. Ein Beschädigen oder Durchdrücken der Grundschicht, verursacht durch die Kontaktstelle des Kabels mit dem leitenden Element, soll dabei vermieden werden.

Diese Aufgabe wird erfindungsgemäß dadurch gelöst, daß das elektrisch leitende Element aus einer dünnen Metallfolie besteht, die zusammen mit dem abisolierten Ende in der Grundschicht eingebettet ist, sowie daß Bereiche vorhanden sind, in welchen die Klebeschicht der Deckschicht zur Grundschicht durchdringt. Die extrem geringe Bauhöhe der Elektrode und die damit verbundene Anschmiegsamkeit resultieren daraus, daß für die Dicke der Elektrode praktisch nur die Grundschicht von Bedeutung ist, in welche das elektrisch leitende Element und das abisolierte Ende des Kabels eingebettet sind. Die Deckschicht trägt ebenfalls nur in geringem Ausmaß zur Dicke der Elektrode bei. Das elektrisch leitende Element in Form einer dünnen Metallfolie sorgt für eine homogene Stromverteilung über die gesamte Elektrodenfläche. Dadurch, daß zumindest Randbereiche der Elektrode vorhanden sind, wo die Klebeschicht von der der Hautoberfläche abgewandten Seite der Elektrode bis zur Grundschicht durchdringt, wird die Metallfolie - eingebettet in die Grundschicht - durch die Klebeschicht fixiert und gleichzeitig an der Grundschicht abgewandten Seite isoliert. Einen weiteren Vorteil stellt die feste Verbindung des Kabels mit dem leitenden Element dar, beispielsweise durch Anlöten, wodurch alle Bauteile entfallen, welche in ihrer Ausdehnung über die Dicke der Elektrode hinausragen. Ein Durchdrücken der Elektrode an der Kontaktstelle mit dem Strom zu- bzw. abführenden Kabel ist somit nicht möglich. Auch bei Elektroden mit kleinem Durchmesser, verglichen mit herkömmlichen Elektroden, bei gleicher Zugbelastung am Kabel, eine wesentlich geringere Hebelwirkung zu erwarten.

Bei einer besonders vorteilhaften Ausgestaltung der Erfindung ist vorgesehen, daß insbesondere bei großflächigen Elektroden das leitende Element Öffnungen aufweist, durch welche die Klebeschicht der Deckschicht durchdringt. Die Anzahl und die Form der Öffnungen im leitenden Element, wobei z.B. aus einer Metallfolie Öffnungen ausgestanzt werden, ist so gewählt, daß eine ausreichende Fixierung bei möglichst homogener Stromverteilung gewährleistet ist. Insbesondere bei größeren quadratischen oder rechteckigen Elektroden muß dafür gesorgt werden, daß auch in den Eck-und Randbereichen ein homogener Stromfluß vorliegt. Dabei kann das elektrisch leitende Element auch gitterförmig ausgebildet sein oder aus einem Drahtgeflecht bestehen.

In einer weiteren Ausgestaltung der Erfindung ist vorgesehen, daß das Kabel mit einem Teil seiner Isolierung in der Grundschicht eingebettet und von der Klebeschicht der Deckschicht fixiert ist, wodurch neben einer weiteren Fixierung des Kabels an der Elektrode gleichzeitig auf einfache Weise für eine ausreichende Isolierung an dieser Stelle gesorgt ist.

Schließlich ist nach einer weiteren Ausgestaltung der Erfindung vorgesehen, daß die Klebeschicht und die Deckschicht durch ein beschriftbares Klebeband realisiert sind. Damit ist ein besonders einfacher Aufbau der Elektrode und eine besonders wirtschaftliche Fertigung gegeben. Es ist auch möglich, die Klebeschicht des Klebebandes selbst leitend auszuführen und gegebenenfalls die Metallfolie in diese Schicht einzubetten, wodurch man einen noch gleichmäßigeren Stromfluß erreicht. Auf der beschriftbaren Deckschicht können Anmerkungen betreffend den Patienten oder den Behandlungsablauf festgehalten werden.

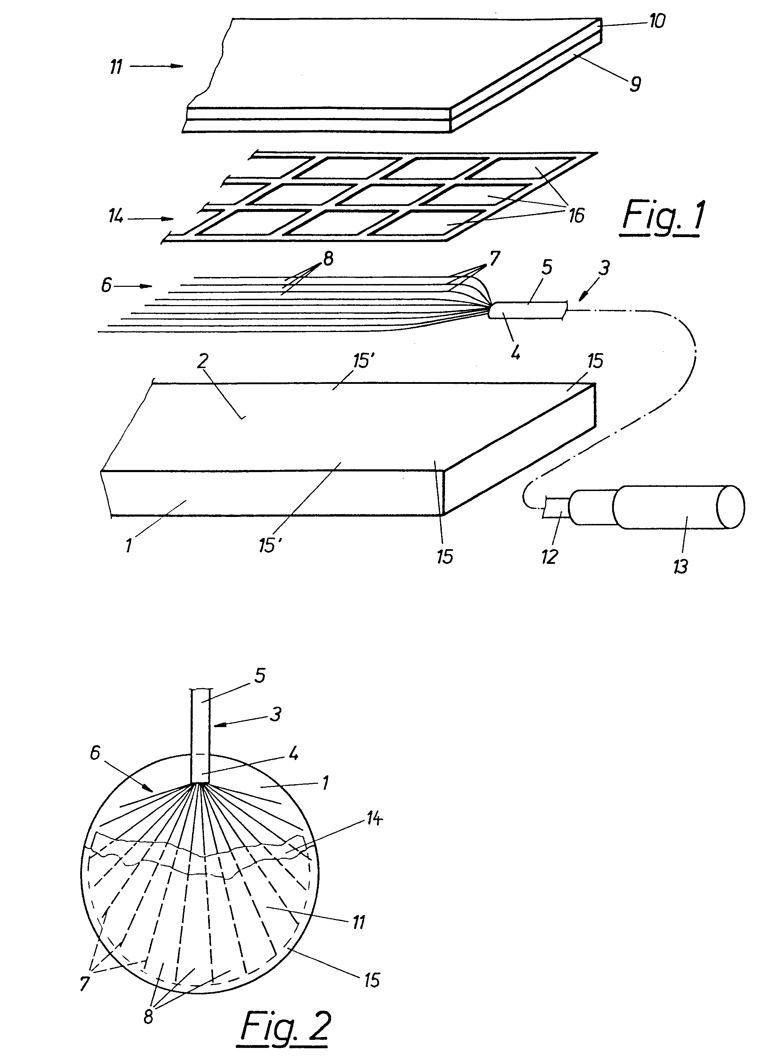

Die Erfindung wird im folgenden anhand der in der Zeichnung dargestellten Ausführungsbeispiele näher erläutert. Es zeigen Fig. 1 schematisch den Aufbau einer erfindungsgemäßen Elektrode in Explosionsdarstellung und Fig. 2 ein anderes Ausführungsbeispiel in Draufsicht.

Die in Fig. 1 dargestellte rechteckige, relativ großflä chige Elektrode, weist eine hautfreundliche, leitende, selbsthaftende Grundschicht 1 auf. Auf der der Hautoberfläche abgewandten Seite 2 dieser Grundschicht 1 liegt ein Kabel 3 mit einem Teil 4 seiner Isolierung 5 auf, wobei das abisolierte Ende 6 mit seinen Drahtlitzen 7 auf der Grundschicht 1 aufliegt oder in diese eingebettet ist. Zwischen der Grundschicht 1, auf welcher das Ende 6 des Kabels 3 aufliegt, und einer Klebeschicht 9 befindet sich das gitterförmige leitende Element 14, durch welches eine homogenere Stromverteilung, bis in die Eckbereiche 15 oder Randbereiche 15' der Elektrode gewährleistet ist. Dieses gitterförmige Element 14 kann durch ein Drahtgeflecht oder durch eine Folie mit ausgestanzten Öffnungen 16 realisiert sein, wobei die Öffnungen 16 genügend groß für ein ausreichendes Durchdringen der Klebeschicht 9 zur Grundschicht 1 ausgeführt sein müssen. Zwischen den einzelnen Litzen 7 befinden sich Bereiche 8, in welchen der direkte Kontakt der über dem elektrisch leitenden Element 14 aufgebrachten Klebeschicht 9 mit der Grundschicht 1 durch die Öffnungen 16 ermöglicht wird. Außerdem wird das Kabel 3 auch im Teil 4 seiner Isolierung 5 von der Klebeschicht 9 erfaßt, was zusätzlich zur Fixierung des Kabels 3 beiträgt bzw. eine ausreichende Isolierung an dieser Stelle garantiert.

Über der Klebeschicht 9 ist eine beschriftbare Deckschicht 10 angeordnet. Diese beiden Schichten 9, 10 können auch durch ein Klebeband 11 realisiert sein. Am von der Elektrode abgewandten Ende 12 des Kabels 3 ist eine Steckverbindung 13, direkt zu einem hier nicht dargestellten Therapiegerät oder zu einem Verlängerungskabel vorgesehen.

Bei der in Fig. 2 dargestellten, kreisförmigen Elektrode mit kleinerem Durchmesser, sind der Darstellung in Fig. 1 entsprechende Teile mit gleichen Bezugzeichen versehen. Das Klebeband 11 wurde zum Teil weggelassen, um die Metallfolie 14 und die Einleitung des Kabels 3 sichtbar zu lassen. Aufgrunde der kleineren Fläche der Elektrode weist hier das leitende Element keine Öffnungen auf, die Durchklebung des Klebebandes 11 erfolgt nur in den Randbereichen 15'.

Deutlich sichtbar ist hier der Bereich 4 der Isolierung 5, welcher auf der Grundschicht 1 aufliegt und von der Klebeschicht des Klebebandes 11 fixiert wird.

**Patentansprüche**

1. Elektrode für Elektrotherapie, mit einer leitenden, selbsthaftend an der Hautoberfläche eines Patienten anbringbaren Grundschicht, mit einem Kabel, dessen abisoliertes Ende mit einem auf der Grundschicht aufliegenden, elektrisch leitenden Element verbunden ist, sowie mit einer Deckschicht, **dadurch gekennzeichnet,** daß das elektrisch leitende Element (14) aus einer dünnen Metallfolie besteht, die zusammen mit dem abisolierten Ende (6) in der Grundschicht (1) eingebettet ist, sowie daß Bereiche vorhanden sind, in welchen die Klebeschicht (9) der Deckschicht (10) zur Grundschicht (1) durchdringt.

2. Elektrode nach Anspruch 1, dadurch gekennzeichnet. daß insbesondere bei großflächigen Elektroden das leitende Element (14) Öffnungen (16) aufweist, durch welche die Klebeschicht (9) der Deckschicht (10) durchdringt.

3. Elektrode nach Anspruch 1 oder 2. dadurch gekennzeichnet. daß das Kabel (3) mit einem Teil (4) seiner Isolierung (5) in der Grundschicht (1) eingebettet und von der Klebeschicht (9) der Deckschicht (10) fixiert ist.

4. Elektrode nach einem der Ansprüche 1 bis 3, dadurch gekennzeichnet, daß die Klebeschicht (9) und die Deckschicht (10) durch ein beschriftbares Klebeband (11) realisiert sind.

0226568

_Fig. 1_

_Fig. 2_

## EINSCHLÄGIGE DOKUMENTE

EP 86890281.8

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | <u>EP - A1 - 0 012 402</u> (MEDTRONIC)<br><br>* Seite 2, Zeile 29 - Seite 3, Zeile 11; Fig. 1-3 *<br><br>-- | 1-3 | A 61 N 1/04 |
| X | <u>EP - A1 - 0 085 327</u> (MEDTRONIC)<br><br>* Seite 12, Zeilen 7-16, 28-30; Fig. 1-3 *<br><br>-- | 1-3 | |
| X | <u>DE - A1 - 2 552 197</u> (KAUFMAN)<br><br>* Seite 7, Zeilen 2-6; Seite 7, Zeile 19 - Seite 8, Zeile 3; Fig. 1,2 *<br><br>-- | 1-3 | |
| D,Y | <u>US - A - 4 125 110</u> (HYMES)<br><br>* Spalte 3, Zeilen 18-31; Fig. 1a,1b *<br><br>-- | 1 | |
| D,Y<br>A | <u>EP - A1 - 0 002 059</u> (MEDTRONIC)<br><br>* Zusammenfassung; Seite 3, Zeilen 31-36; Seite 6, Zeilen 3-21; Fig. 1-3 *<br><br>-- | 1<br>3 | RECHERCHIERTE SACHGEBIETE (Int. C. 4)<br><br>A 61 N<br>A 61 B |
| A | <u>EP - A1 - 0 029 245</u> (SIEMENS)<br><br>* Zusammenfassung; Seite 6, Zeile 17 - Seite 7, Zeile 16; Fig. 3,4 *<br><br>-- | 1-3 | |
| A | <u>EP - A1 - 0 097 436</u> (MINNESOTA MINING)<br><br>* Seite 4, Zeile 20 - Seite 5, Zeile 9; Fig. 1,2 *<br><br>-- | 1-3 | |

Der vorliegende Recherchenbericht wurde für alle Patentansprüche erstellt.

| Recherchenort | Abschlußdatum der Recherche | Prüfer |
|---|---|---|
| WIEN | 27-03-1987 | NEGWER |

Europäisches
Patentamt

**EUROPÄISCHER RECHERCHENBERICHT**

Nummer der Anmeldung

**EINSCHLÄGIGE DOKUMENTE**

EP 86890281.8

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| A | FR - A1 - 2 449 454 (ROMEO)<br><br>* Seite 2, Zeile 28 - Seite 3, Zeile 5; Fig. 1-3 *<br><br>---- | 1-3 | |
| | | | **RECHERCHIERTE SACHGEBIETE (Int. Cl. 4)** |

Der vorliegende Recherchenbericht wurde für alle Patentansprüche erstellt.

| Recherchenort | Abschlußdatum der Recherche | Prüfer |
|---|---|---|
| WIEN | 27-03-1987 | NEGWER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82